# EUROPEAN PATENT SPECIFICATION

(11) **EP 3 145 490 B1**
(45) Date of publication and mention of the grant of the patent: **28.03.2018**
(21) Application number: 15728175.9
(22) Date of filing: 05.05.2015
(51) Int. Cl.: A61K 9/127, A61K 31/202, A61K 31/353, A61P 17/14

(54) **LIPOSOMES CONTAINING DI-HOMO-GAMMA LINOLENIC ACID (DGLA), FORMULATIONS CONTAINING THEM AND USE THEREOF**
LIPOSOMEN MIT DI-HOMO-GAMMA LINOLENSÄURE (DGLA), FORMULIERUNG DAMIT UND VERWENDUNG DAVON
LIPOSOMES CONTENANT UN ACIDE LINOLÉNIQUE DI-HOMO-GAMMA (DGLA), FORMULATIONS LES CONTENANT, ET LEUR UTILISATION

(30) Priority: 06.05.2014 IT CA20140003
(43) Date of publication of application: 29.03.2017
(73) Proprietor: Fidia Farmaceutici S.p.A., 35031 Abano Terme (PD) (IT)
(72) Inventor: BROTZU, Giovanni, I-09127 Cagliari (CA) (IT); BROTZU, Giuseppe, I-09127 Cagliari (CA) (IT)
(74) Representative: Perani & Partners S.p.A.
(86) International application number: PCT/IB2015/053267
(87) International publication number: WO 2015/170247

(56) References cited:
- EP-A1- 0 309 086
- WO-A1-2011/095938
- WO-A1-2013/171668
- GB-A- 2 150 588
- US-A- 5 962 015
- US-A1- 2003 064 929
- US-A1- 2009 197 954
- NATTAYA LOURITH ET AL: "Hair loss and herbs for treatment", JOURNAL OF COSMETIC DERMATOLOGY, vol. 12, no. 3, 1 September 2013 (2013-09-01), pages 210-222, XP055078947, ISSN: 1473-2130, DOI: 10.1111/jocd.12051

## Description

### Field of the invention

The present invention discloses and claims liposomes comprising active substances, formulated for topical and/or oral use and preferentially for topical use, to be used for the treatment of alopecia, baldness, hair loss, also in postmenopausal women, and in general for hair regrowth.

### State of the art

Hair loss is an extremely common phenomenon concerning both men and women, caused by various factors such as stress, diet, seasonal changes, hereditary factors, particular diseases or pharmacological treatments; eventually, in the female population, it can be caused by the lack of sexual hormones occurring during menopause. In fact, the female menopausal population generally reports, if not hair loss, at least a decrease in the characteristics of strength, brightness, resistance to brushing.

The phenomenon often has such consequences on the patient that it is considered an actual disease, and anyway it has an unquestionable impact from the aesthetic point of view. There are many methods described for treatment and prevention of baldness through aids acting on the hair shaft, trying to make it stronger, or on the bulb, attempting to identify the reasons why it becomes atrophied to find a solution. Some positive attempts were made by employing actual drugs, such as the well known Minoxidil and Finasteride.

They act on completely different districts. Finasteride acts as an inhibitor of type 2 alpha-reductase (involved in the synthesis of steroids), while Minoxidil is a vasodilator. Both products, although quite efficient, display remarkable complications, given their nature of drugs and the inevitable appearance of side effects, and this has strongly limited their use.

Products acting on the hair shaft, and that are essentially of cosmetic type, have proven to be poorly efficient in time.

Despite the multiple remedies available on the market, the problem is far from being called satisfactorily solved. A suggestion comes from WO2013171668, wherein PGE1 is employed in association with plant sterols, and administered locally on the scalp. PGE1 is a drug acting on several levels: it inhibits type 1 and 2 alpha-reductase, is a vasodilator and improves angiogenesis.

Such compositions are delivered in the form of liposomes, which had already demonstrated to be valuable carriers for PGE1 for systemic or local treatment of vascular diseases and their cutaneous outbreaks (ulcers). For example, WO 2011/095938 discloses unilamellar liposomes, encapsulating PGE1 and/or PGE1-α-cyclodextrin in association with L-propionyl-carnitine for the treatment of vascular diseases in diabetic subjects.

Encapsulation in liposomes is not able to increase the very poor stability of PGE1, though; the findings of WO2013171668 in fact, must be freeze-dried or, if in suspension, stored at low temperature (about -20°C) up to the time of use, if one intends to prevent the almost complete oxidation of PGE1. This obviously represents a complication not only on the industrial production level, but mainly on the product safety level. To act against the oxidative process, in fact, the trend is to resort to PGE1 doses that might be dangerous for the body, considered the repeated employment of these products, which must be administered over very long periods.

### SUMMARY OF THE INVENTION

The present invention overcomes the limits of the state of the art through liposome formulations containing the PGE1 precursor dihomo-gamma-linolenic acid stable at room temperature, storable in a liquid, semi-solid or solid form, directly applicable without preventive preparation and absolutely free of any kind of side effect related to the use of an actual drug. Clinical trials that were carried out further demonstrated a surprisingly higher efficacy of the products described herein in comparison with what is known, so as to make their use possible at concentrations markedly lower than those of already known products.

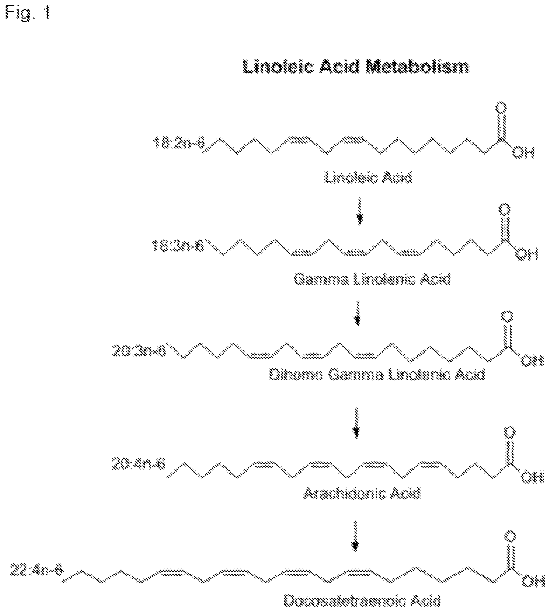

### Description of figure 1

Figure 1 reports the synthetic scheme of linoleic acid metabolism.

### Detailed description of the invention

Object of the present invention is liposomes comprising active substances, formulated for topical and/or oral use and preferentially for topical use, to be used for the treatment of alopecia, baldness, hair loss, also in postmenopausal women, and in general for hair regrowth. Substances enclosed in the liposomes are the PGE1 precursor dihomo-gamma-linolenic acid (DGLA) in association with the plant estrogen equol and a compound able to increase cation capacity of the liposome, then improving its adhesiveness. Liposomes further comprise a stabilizer, to improve stability of the preparations. Preferably used for the purposes of the present invention.

Gamma-linolenic acid and dihomo-gamma-linolenic acid are sometimes defined as gamma-linoleic, and dihomo-gamma-linoleic acid, respectively, due to the fact that they are intermediates in the process of prostaglandin biosynthesis starting from linoleic acid, which is progressively desaturated according to the scheme reported in figure 1.

For the purposes of the present invention, the definitions "gamma-linolenic" acid and "gamma-linoleic acid", are synonyms and identify one single compound reported in figure 1, similarly, the definitions "dihomo-gamma-linoleic acid" and "dihomo-gamma-linolenic acid", both identifying one single chemical compound reported in figure 1 too, are also synonyms, for a compound that in the present description is also identified by the acronym DGLA.

Doses of DGLA used range between 0.05% and 0.3% and preferably between 0.1% and 0.2% of the phospholipid amount used.

Liposomes according to the invention are composed of a phospholipid vesicle containing an aqueous solution core. Phospholipids composing the vesicle wall can be natural phospholipids or can be of synthetic origin, considering their high biocompatibility and absence of toxicity.

Phospholipids that can be used according to the invention are, for example: phosphatidylcholine (lecithin), phosphatidylethanolamine, phosphatidylserine, phosphatidylglycerol, phosphatidylinositol, dimyristoylphosphatidylcholine (DPMC), dipalmitoylphosphatidylcholine (DPPC), distearoylphosphatidylcholine (DSPC), palmitoyl-stearoylphosphatidylcholine, sphingomyelin, and other similar ones. Particularly suitable are phosphatidylcholine, dipalmitoylphosphatidylcholine (DPPC), dimyristoylphosphatidylcholine (DPMC) and preferentially phosphatidylcholine. The plant estrogen used is equol, a key metabolite of daidzein, for long already in use by oral route for reducing menopausal symptoms (flushes, sweating, sudden mood changes). - Doses employed range between 0.1% and 2%, and preferably between 0.3% and 1% of phospholipid amount.

To increase the adhesiveness of liposomes and to promote cell metabolism of the endothelium of micro-capillaries, compounds able to increase cation capacity of the liposome are also used and selected from carnitine, and its derivative L-propionylcarnitine, which, in addition, is a carrier for fatty acids and allows mitochondria to use them for ATP production. The amount of L-propionylcarnitine used ranges between 0.1% and 2%, and preferably between 0.3% and 1.3% of phospholipid amount.

Liposomes comprise stabilizers selected from cholesterol, cholesterol sulphate, stearylamine. Within the scope of the present invention the use of stearylamine that, in addition to stabilizing the microemulsion increases adhesiveness of liposomes and further increases their cation capacity, was shown to be particularly useful. Various laboratory tests demonstrated that the percentage of stearylamine used is able to influence the size of liposomes and their charge more considerably than other substances used. Stability tests additionally highlighted that liposomes prepared with stearylamine at concentrations higher than 0.5%, preferably between 1% and 5%, and even more preferably between 2% and 4% of phospholipid, in particular phosphatidylcholine, retain their peculiar characteristics unchanged for at least 30 days.

Object of the present invention is thus phospholipid liposomes comprising:
- the PGE1 precursor dihomo-gamma-linolenic acid (DGLA),
- the plant estrogen equol, preferably S-equol,
- a compound able to increase cation capacity of the liposome selected from carnitine and its derivative L-propionylcarnitine, a stabilizer selected from cholesterol, cholesterol sulphate and stearylamine,
for use in the treatment of alopecia, baldness, hair loss.

More preferably liposomes according to the present invention are composed of phosphatidylcholine and comprise dihomo-gamma-linoleic acid at a concentration ranging from 0.05% to 0.3% and preferably from 0.1% to 0.2% of the phospholipid amount used, equol, even more preferably S-equol, at a concentration comprised between 0.1% and 2% and preferably between 0.3% and 1% of the phospholipid amount used, L-propionylcarnitine at a concentration comprised between 0.1% and 2% and preferably between 0.3% and 1.3% of the phospholipid amount used, stearylamine at a concentration comprised between 1% and 5% and more preferably between 2% and 4% of the phospholipid amount used.

Liposomes according to the invention can be readily prepared (method A)
- admixing solutions of the various components in an organic solvent (selected from ethanol, methanol, isopropanol, acetone or mixtures thereof) and water and
- sonicating the thus obtained mixture for the time needed.

The preferably selected organic solvent is ethanol.

Alternatively, it is possible to (method B)
- sonicate the mixtures comprised of the solutions of the various components in an organic solvent only (selected from ethanol, methanol, isopropanol, acetone or mixtures thereof)
- evaporate the organic solvent
- take up in an aqueous solution.

Also in this case the preferably used solvent is ethanol.

Additionally, to increase the adherence of liposomes to the cells of the dermis avoiding their traumatic removal, the outer surface of the liposomes may be optionally coated with hydrophilic polymers, such as for example polylisine, polyornithine, fibronectin and/or mixtures thereof.

Such coating, if applied, can be very simply obtained according to known techniques, for example letting the mixture of liposomes drop into a solution of the selected polymer.

Further object of the present invention are pharmaceutical compositions or formulations comprising the liposomes of the invention and suitable for topical application and/or for oral administration.

Formulations for topical use according to the invention comprising liposomes as described above are normally in classic forms for topical application, such as for example: aqueous suspensions (lotions), ointments, creams, gels, sprays, polymeric films, in which liposomes are dispersed using known methods of pharmaceutical technique for preparing said formulations. Eventually, topical administration of liposome suspensions described herein is also possible in the form of aerosols, after producing the specific pharmaceutical form according to known techniques.

Gels and polymeric films can be made of organic polymers such as for example: polyacrilates, sodium hyaluronate, hydroxypropylcellulose (HPMC), polyethylene glycol 400 (PEG 400) and water, in suitable ratios, taken individually or in mixtures thereof, and are characterized in terms of viscoelastic properties, thickness and *in vitro* bioadhesion using a rheometer, a micrometer and a tensiometer respectively, according to the methods known to the expert in the field.

Films are used for preparing dressings for topical application and are comprised, for example, of strips of various sizes for application to the skin. Such films are particularly useful when the area to be treated is very wide, and thus administering the product as a lotion, cream or gel becomes less comfortable.

Lotions, eventually, can also be dispensed from "spray no-gas", then propellant free, bottles.

Additionally, it is possible to administer liposomes by oral route after having them properly delivered in capsules of gelatine or other substances suitable for the purpose and known to the expert in the field.

Regardless of the selected pharmaceutical form, it is clear that all excipients and all tactics known to the technical formulator in order to ensure stability, storability, absence of contamination, etc. of the preparations will be employed.

The present invention relates to pharmaceutical compositions for the treatment of alopecia, baldness, hair loss, also in postmenopausal women, and in general to promote hair regrowth, such compositions comprise phospholipid liposomes comprising:
- the PGE1 synthesis precursor dihomo-gamma-linolenic acid (DGLA),
- the plant estrogen equol, preferably S-equol,
- a compound able to increase the cation capacity of the liposome selected from carnitine and its derivative L-propionylcarnitine,
- a stabilizer selected from cholesterol, cholesterol sulphate and stearylamine and containing pharmaceutically acceptable excipients.

Preferably the pharmaceutical compositions described herein comprise phospholipid liposomes composed of phosphatidylcholine, dipalmitoylphosphatidylcholine (DPPC), dimyristoylphosphatidylcholine (DPMC) and preferably phosphatidylcholine, and
- DGLA, at a concentration ranging from 0.05% to 0.3% and preferably from 0.1% to 0.2% of the phospholipid amount used;
- equol, at a concentration ranging from 0.1% to 2% and preferably from 0.3% to 1% of the phospholipid amount used;
- L-propionylcarnitine at a concentration comprised between 0.1% and 2% and preferably between 0.3% and 1.3% of the phospholipid amount used;
- stearylamine at a concentration comprised between 1% and 5% and more preferably between 2% and 4% of the phospholipid amount used and suitable pharmaceutically acceptable excipients.

The invention is now better decsribed by the following examples.

### Example 1: preparation of a liposome mixture in the form of a lotion comprising 1% stearylamine (method A)

| | |
|---|---|
| Phosphatidylcholine (Lipid S75 Humangrade) | 1 g |
| DGLA | 1.25 mg |
| S-Equol | 7 mg |
| Ethanol | 1 ml |
| L-propionylcarnitine | 7 mg |
| Stearylamine | 10 mg |
| Sterile water | 5 ml |

DGLA, S-equol and stearylamine are dissolved in the dose of ethanol and phosphatidylcholine is added to the solution. Propionylcarnitine is dissolved in 5 ml of water and the thus obtained solution is added to the previous one. The resulting mixture is placed in a sonicator (Sonipress 150 kw) and submitted to 25 sonication cycles. Each cycle is composed of 5 seconds of full power sonication alternating with 2 seconds of rest. The lotion thus obtained is divided into 5 vials and each one is brought to the volume of 7 ml with additional sterile water. The lotion thus obtained is ready for use.

### Example 2: preparation of a liposome mixture in the form of a lotion comprising 1% stearylamine (method B)

| | |
|---|---|
| Phosphatidylcholine (Lipid S75 Humangrade) | 1 g |
| DGLA | 1.25 mg |
| S-Equol | 7 mg |
| Ethanol | 1 ml |
| L-propionylcarnitine | 7 mg |
| Stearylamine | 10 mg |
| Sterile water | 5 ml |

DGLA, S-equol, stearylamine and L-propionylcarnitine are dissolved in the dose of ethanol and the solution obtained, after addition of phosphatidylcholine, is placed in a sonicator (Sonipress 150 kw) and submitted to 25 sonication cycles. Each cycle is composed of 5 seconds of full power sonication alternating with 2 seconds of rest. Ethanol is then completely evaporated and the liposomes obtained are brought into contact with 5 ml of sterile water. The lotion thus obtained is divided into 5 vials and each one is brought to the volume of 7 ml with additional sterile water. The lotion is ready for use.

### Example 3: preparation of a liposome mixture in the form of a lotion comprising 2% stearylamine (method A)

See example 1, with the only variation of the dose of stearylamine employed, equal to 20 mg.

### Example 4: preparation of a liposome mixture in the form of a lotion comprising 3% stearylamine (method A)

See example 1, with the only variation of the dose of stearylamine employed, equal to 30 mg.

To increase their shelf-life, the lotions thus prepared can also be freeze-dried according to methods known by the expert in the field, and reconstituted with water at the time of application.

### Characterization of liposomes

Liposomes comprising various percentages of stearylamine are characterized in terms of size, polydispersity index (PI) and zeta potential (Pζ); PI is measured via Photon Correlation Spectroscopy (PCS) and expresses the size uniformity of the particles constituting the dispersed system. The lower the PI value, the higher the size uniformity of the particles considered and then, the higher the homogeneity of the dispersion.

As a convention, a system has high uniformity for PI values of <1.1.

The zeta potential (Pζ) expresses the electrophoretic mobility of particles (in this case, liposomes) in a thermostated cell and it is representative of the stability of the dispersed systems; it was measured through M3-PALS (Phase Analysis Light Scattering) with the Zetasizer nano instrument (Malvern Instrument, UK). It should be remembered that Pζ values higher than -30 mV are an index of stability.

Morphological-structural characteristics were studied through transmission electron microscopy (TEM) and polarized light optical microscopy.

The diameter of liposomes results to be, regardless of the amount of stearylamine employed, always lower than 100 nm and the polydispersity index lower than 0.2. Pζ values demonstrate that the preparations are stable. Samples A, B and C were prepared according to examples 1, 3, and 4 respectively, and after detection of the characteristics, were stored for 30 days at room temperature. At the end, a further detection of Pζ values was performed and data are summarized in the following table 1.

**Table 1**

| Samples | Diameter (nm) | PI | Pζ (mVolt) (starting) | Pζ (mVolt) (30 d) |
|---|---|---|---|---|
| A (1% stearylamine) | 74.84±0.88 | 0.187 | -24.7 | -30.05 |
| B (2% stearylamine) | 93.52±1.17 | 0.150 | +24.9 | +8.52 |
| C (3% stearylamine) | 96.02±1.12 | 0.178 | +42.9 | +23.5 |

It is clear that the samples prepared are all homogeneous, and this ensures uniformity of distribution of the active substances, and stability, both just prepared and, mainly and surprisingly, even after 30 days after preparation. Such observations are true for all the concentrations of stearylamine used, and particularly for sample C, which contains it at a concentration of 3%.

Encapsulation efficiency (E%) of DGLA and plant estrogen in liposomes was determined by HPLC, after carrying out a rupture of the liposomes with a suitable membrane lysating agent, such as for example Triton X-100. The amount of active substances present after purification is in the order of 85% of the starting concentration and remains the same also after 30-day storage.

### Experimental study

Patients of female and male gender characterized by massive hair reduction due to various causes, and also a small number of patients of female gender of postmenopausal age, reporting hair thinning, hair reduction and loss of hair strength were selected. The clinical efficacy of preparation 4 that on previous tests displayed the best characteristics of homogeneity and stability was evaluated.

### Treatments:

Lotion A, prepared according to the present example 4;
Lotion B, comparison, prepared as described in WO 2011/095938 (example 1) and then containing PGE1 and S-equol in 1:1 ratio
Lotion C, containing 7 mg of PGE1 enclosed in liposomes as the only active substance, prepared according to method A;
Lotion D, containing 1,25 mg of DGLA enclosed in liposomes as the only active substance, prepared according to method A.

### Groups of patients:

- Group 1: 10 (6 male, 4 female) patients treated for 120 days with 1 ml/day of Lotion A to be applied on a well defined area of the scalp;
- Group 2: 10 (5 male, 5 female) patients treated for 120 days with 1 ml/day of Lotion B to be applied on a well defined area of the scalp;
- Group 3: 6 female menopausal patients for about 3 years, without specific diseases related to hair reduction, treated for 120 days with 1 ml/day of Lotion A to be applied on a well defined area of the scalp.
- Group 4: 4 (2 male, 2 female) patients treated for 120 days with 1 ml/day of Lotion C to be applied on a well defined area of the scalp;
- Group 5: 4 (2 male, 2 female) patients treated for 120 days with 1 ml/day of Lotion D to be applied on a well defined area of the scalp;

Evaluation was carried out at pre-determined time points by dermoscopy and direct observation with photographic detections.

### Results

On day 7: both group 1 and group 2 experience a reduction of hair loss, defined "substantial" by patients of group 1 and "modest" by those of group 2. Group 3 feels hair as more robust. Groups 4 and 5 do not report any variation;

On day 20: in group 1 all the patients have stopped losing hair and report the presence of a substantial fuzz. In group 2 hair loss has stopped in 6 patients and fuzz, where present, is more modest (in terms of number/cm²). Group 3 reports an aesthetic improvement related to hair brightness. Group 4 reports a minimal reduction in hair loss. Group 5 does not report any variation;

On day 45: in group 1, fuzz has grown markedly stronger and takes the colour and consistency of natural hair. In group 2, as expected, all the patients have stopped losing hair, but only some of them report a certain degree of regrowth, fuzz is very weak and not completely coloured yet. Group 3 reports disappearance of split ends. Group 4 reports arrested hair loss in all the subjects and, in some of them, presence of weak fuzz. Group 5 does not report any variation;

On day 90: in group 1 all the patients report total disappearance of hair reduction areas that have been replaced by robust and shiny hair. In group 2 hair reduction is still visible, even if areas are all covered with thick fuzz. Group 3 confirms a general hair improvement. Groups 4 and 5 do not record variations from the previous evaluation;

On day 120: group 1 confirms results already seen at day 90. Group 2 continues improvement, even if in some patients of male gender the fuzz has not got the characteristics of strength and colour typical of hair yet. Group 3 confirms results previously obtained. Groups 4 and 5 do not record variations from the previous evaluation.

During the whole phase of the study the patients' skin was regularly assayed by dermoscopy, highlighting an improvement of circulation. Additionally, treated skin did not display allergic or inflammatory reactions.

From the observations reported here, first of all, it can be inferred that the formulations object of the present invention are clinically efficient in:
- stopping hair loss;
- promoting hair regrowth in areas with alopecia;
- strengthening and fortifying hair, bringing it back to its original state
both in male and female subjects with forms of hair reduction or actual alopecia due to different causes, and in menopausal women.

Formulations set up here additionally:
- do not contain potentially toxic substances or that may interfere with other active ingredients;
- are not irritating for the skin: even after long term application no evidence of irritation or inflammation was reported;
- are stable in time: analysis carried out on the content of the vials 30 days after preparation detects
- significant Pζ values in terms of stability, particularly for formulations containing 2% or, better, 3% stearylamine
- an unchanged content of active substances enclosed in liposomes;
- can be stored at room temperature and in a ready-to-use form;
   and, surprisingly
- act more quickly and more effectively: comparison tests clearly demonstrate that group 1 has a more rapid, more substantial improvement and, mainly, it involves all the patients as compared with group 2, treated with Lotion B.

Again, it should be recalled that Lotion B contains PGE1 and equol in 1:1 ratio, while Lotion A contains 30% less of active substance (DGLA). This means that the findings of the present invention exert an unexpectedly superior effect than what known, at markedly lower concentrations of active substances.

These results should be attributed only to the formulation set up herein. In fact, it is clear from data analysis that patients treated with PGE1 alone, even at the concentrations described in WO2011/095938, experience a very modest variation of the situation. Still different and most important is the observation of group 5, treated with DGLA alone at the same concentration employed in Lotion A: there is no reduction in hair loss and even less any regrowth, not even of fuzz.

The extraordinary effects that are displayed by the compositions of the present invention are then due to synergies between single components, promoted by the characteristics of the liposome suspensions used.

## Claims

1. Phospholipid liposomes comprising dihomo-gamma-linolenic acid (DGLA) at a concentration ranging from 0.05% to 0.3% of the phospholipid amount used, the plant estrogen equol at a concentration comprised between 0.1% and 2% of the phospholipid amount used; a compound able to increase cation capacity of the liposome selected from carnitine and its derivative L-propionylcarnitine; a stabilizer selected from cholesterol, cholesterol sulphate and stearylamine.

2. The phospholipid liposomes according to claim 1, wherein the phospholipids are selected from phosphatidylcholine, phosphatidylethanolamine, phosphatidylserine, phosphatidylglycerol, phosphatidylinositol, dimyristoylphosphatidylcholine (DPMC), dipalmitoylphosphatidylcholine (DPPC), distearoylphosphatidylcholine (DSPC), palmitoyl-stearoylphosphatidylcholine, sphingomyelin and, preferably, are selected from phosphatidylcholine, dipalmitoylphosphatidylcholine (DPPC), dimyristoylphosphatidylcholine (DPMC) and even more preferably from phosphatidylcholine.

3. The phospholipid liposomes according to claim 1 or 2, comprising DGLA at a concentration ranging from 0.05% to 0.3% and preferably from 0.1% to 0.2% of the phospholipid amount used.

4. The phospholipid liposomes according to any one of claims 1-3, comprising equol at a concentration comprised between 0.1% and 2% and preferably between 0.3% and 1% of the phospholipid amount used.

5. The phospholipid liposomes according to any one of claims 1-4, wherein said compound able to increase the cation capacity of the liposome is L-propionylcarnitine at a concentration comprised between 0.1% and 2%, and preferably between 0.3% and 1.3% of the phospholipid amount used.

6. The phospholipid liposomes according to any one of claims 1-5 wherein said stabilizer is stearylamine at a concentration comprised between 1% and 5% and more preferably between 2% and 4% of the phospholipid amount used.

7. The phospholipid liposomes according to any one of claims 1-6, on the external surface of which hydrophilic polymers selected from polylisine, polyornithine, fibronectin and/or mixtures thereof are present.

8. The phospholipid liposomes according to any one of claims 1-7 comprising: phosphatidylcholine DGLA at a concentration ranging from 0.05% to 0.3% and preferably from 0.1% to 0.2% of the phospholipid amount used, equol at a concentration comprised between 0.1% and 2% and preferably between 0.3% and 1% of the phospholipid amount used, L-propionylcarnitine at a concentration comprised between 0.1% and 2% and preferably between 0.3% and 1.3% of the phospholipid amount used, stearylamine at a concentration comprised between 1% and 5% and more preferably between 2% and 4% of the phospholipid amount used, and further containing pharmaceutically acceptable excipients.

9. Phospholipid liposomes according to any one of claims 1-8 for use in the treatment of alopecia, baldness, hair loss.

10. Pharmaceutical compositions comprising liposomes according to any one of claims 1 to 8, in association with suitable pharmaceutically acceptable excipients and/or diluents.

11. The pharmaceutical compositions according to claim 10 suitable for oral and/or topical administration.

12. The pharmaceutical compositions according to claims 10-11 for topical application in the form of aqueous suspensions (lotions), ointments, creams, gels, sprays, polymeric films.

13. The pharmaceutical compositions according to claims 10-11 for oral administration.

## Patentansprüche

1. Phospholipid-Liposomen, umfassend Dihomo-gamma-linolensäure (DGLA) in einer Konzentration im Bereich von 0,05% bis 0,3% der eingesetzten Phospholipidmenge, das Pflanzenöstrogen Equol in einer Konzentration zwischen 0,1% und 2% der eingesetzten Phospholipidmenge; eine Verbindung, die in der Lage ist, die Kationenkapazität des aus Carnitin und seinem Derivat L-Propionylcarnitin ausgewählten Liposoms zu erhöhen; einen Stabilisator, ausgewählt aus Cholesterin, Cholesterolsulfat und Stearylamin.

2. Phospholipid-Liposomen nach Anspruch 1, wobei die Phospholipide aus Phosphatidylcholin, Phosphatidylethanolamin, Phosphatidylserin, Phosphatidylglycerol, Phosphatidylinositol, Dimyristoylphosphatidylcholin (DPMC), Dipalmitoylphosphatidylcholin (DPPC), Distearoylphosphatidylcholin (DSPC), Palmitoylstearoylphosphatidylcholin, Sphingomyelin ausgewählt werden und vorzugsweise aus Phosphatidylcholin, Dipalmitoylphosphatidylcholin (DPPC), Dimyristoylphosphatidylcholin (DPMC) und noch bevorzugter aus Phosphatidylcholin ausgewählt werden.

3. Phospholipid-Liposomen nach Anspruch 1 oder 2, umfassend DGLA in einer Konzentration im Bereich von 0,05% bis 0,3% und vorzugsweise von 0,1% bis 0,2% der eingesetzten Phospholipidmenge.

4. Phospholipid-Liposomen nach einem der Ansprüche 1 bis 3, umfassend Equol in einer Konzentration zwischen 0,1 % und 2% und vorzugsweise zwischen 0,3% und 1 % der eingesetzten Phospholipidmenge.

5. Phospholipid-Liposomen nach einem der Ansprüche 1 bis 4, wobei die Verbindung, die die Kationenkapazität des Liposoms erhöhen kann, L-Propionylcarnitin in einer Konzentration zwischen 0,1 % und 2% und vorzugsweise zwischen 0,3% und 1,3% der eingesetzten Phospholipidmenge ist.

6. Phospholipid-Liposomen nach einem der Ansprüche 1 bis 5, wobei der Stabilisator Stearylamin in einer Konzentration zwischen 1% und 5% und vorzugsweise zwischen 2% und 4% der eingesetzten Phospholipidmenge ist.

7. Phospholipid-Liposomen nach einem der Ansprüche 1 bis 6, auf deren äußerer Oberfläche aus Polylisin, Polyornithin, Fibronectin und/oder Mischungen davon ausgewählte hydrophile Polymere vorliegen.

8. Phospholipid-Liposomen nach einem der Ansprüche 1 bis 7, umfassend Phosphatidylcholin DGLA in einer Konzentration im Bereich von 0,05 bis 0,3% und vorzugsweise von 0,1 bis 0,2% der eingesetzten Phospholipidmenge, Equol in einer Konzentration zwischen 0,1% und 2% und vorzugsweise zwischen 0,3% und 1% der eingesetzten Phospholipidmenge, L-Propionylcarnitin in einer Konzentration zwischen 0,1% und 2% und vorzugsweise zwischen 0,3% und 1,3% der eingesetzten Phospholipidmenge, Stearylamin in einer Konzentration zwischen 1% und 5% und vorzugsweise zwischen 2% und 4% der eingesetzten Phospholipidmenge, und die ferner pharmazeutisch annehmbare Hilfsstoffe enthalten.

9. Phospholipid-Liposomen nach einem der Ansprüche 1 bis 8 zur Verwendung bei der Behandlung von Alopezie, Kahlheit, Haarausfall.

10. Pharmazeutische Zusammensetzungen, umfassend Liposomen nach einem der Ansprüche 1 bis 8, in Verbindung mit geeigneten pharmazeutisch annehmbaren Hilfsstoffen und/oder Verdünnungsmitteln.

11. Pharmazeutische Zusammensetzungen nach Anspruch 10, die zur oralen und/oder topischen Verabreichung geeignet sind.

12. Pharmazeutische Zusammensetzungen nach den Ansprüchen 10-11 zur topischen Anwendung in Form von wässrigen Suspensionen (Lotionen), Salben, Cremes, Gelen, Sprays, Polymerfolien.

13. Pharmazeutische Zusammensetzungen nach den Ansprüchen 10-11 zur oralen Verabreichung.

## Revendications

1. Liposomes phospholipidiques comprenant de l'acide dihomo-gamma-linolénique (DGLA) à une concentration allant de 0,05% à 0,3% de la quantité de phospholipides utilisée, de l'oestrogène végétal équol à une concentration comprise entre 0,1% et 2% de la quantité de phospholipides utilisée; un composé capable d'augmenter la capacité cationique du liposome choisi parmi la carnitine et son dérivé L-propionylcarnitine; un stabilisant choisi parmi le cholestérol, le sulfate de cholestérol et la stéarylamine.

2. Liposomes phospholipidiques selon la revendication 1, dans lesquels les phospholipides sont choisis parmi la phosphatidylcholine, la phosphatidyléthanolamine, la phosphatidylsérine, le phosphatidylglycérol, le phosphatidylinositol, la dimyristoylphosphatidylcholine (DPMC), la dipalmitoylphosphatidylcholine (DPPC), la distéaroylphosphatidylcholine (DSPC), la palmitoylstéaroylphosphatidylcholine, la sphingomyéline et, de préférence, sont choisis parmi la phosphatidylcholine, la dipalmitoylphosphatidylcholine (DPPC), la dimyristoylphosphatidylcholine (DPMC) et encore plus de préférence de la phosphatidylcholine.

3. Liposomes phospholipidiques selon la revendication 1 ou 2, comprenant du DGLA à une concentration allant de 0,05% à 0,3% et de préférence de 0,1% à 0,2% de la quantité de phospholipide utilisée.

4. Liposomes de phospholipides selon l'une quelconque des revendications 1 à 3, comprenant de l'équol à une concentration comprise entre 0,1 % et 2% et de préférence entre 0,3% de la quantité de phospholipide utilisée.

5. Liposomes phospholipidiques selon l'une quelconque des revendications 1 à 4, dans lesquels ledit composé capable d'augmenter la capacité cationique du liposome est la L-propionylcarnitine à une concentration comprise entre 0,1% et 2%, et de préférence entre 0,3% et 1,3% de la quantité de phospholipide utilisée.

6. Liposomes phospholipidiques selon l'une quelconque des revendications 1 à 5, dans lesquels ledit stabilisant est la stéarylamine à une concentration comprise entre 1% et 5% et plus de préférence entre 2% et 4% de la quantité de phospholipide utilisée.

7. Liposomes de phospholipides selon l'une quelconque des revendications 1 à 6, sur la surface externe desquels il y a des polymères hydrophiles choisis parmi la polylisine, la polyornithine, la fibronectine et/ou leurs mélanges.

8. Liposomes phospholipidiques selon l'une quelconque des revendications 1 à 7 comprenant: phosphatidylcholine DGLA à une concentration allant de 0,05% à 0,3% et de préférence de 0,1 à 0,2% de la quantité de phospholipide utilisée, équol à une concentration comprise entre 0,1% et 2% et de préférence entre 0,3% et 1% de la quantité de phospholipide utilisée, L-propionylcarnitine à une concentration comprise entre 0,1% et 2% et de préférence entre 0,3% et 1,3% de la quantité de phospholipide utilisée, stéarylamine à une concentration comprise entre 1% et 5%, et, plus de préférence, entre 2% et 4% de la quantité de phospholipide utilisée, et contenant en outre des excipients pharmaceutiquement acceptables.

9. Liposomes de phospholipides selon l'une quelconque des revendications 1 à 8 pour une utilisation dans le traitement de l'alopécie, de la calvitie, de la chute des cheveux.

10. Compositions pharmaceutiques comprenant des liposomes selon l'une quelconque des revendications 1 à 8, en association avec des excipients et/ou des diluants pharmaceutiquement acceptables appropriés.

11. Compositions pharmaceutiques selon la revendication 10, appropriées pour l'administration orale et/ou topique.

12. Compositions pharmaceutiques selon les revendications 10-11 pour une application topique sous la forme de suspensions aqueuses (lotions), d'onguents, de crèmes, de gels, de sprays, de films polymères.

13. Compositions pharmaceutiques selon les revendications 10-11 pour l'administration orale.
